# EUROPÄISCHE PATENTANMELDUNG

(11) **EP 2 377 520 A1**
(43) Veröffentlichungstag der Anmeldung: **19.10.2011**
(21) Anmeldenummer: 10157606.4
(22) Anmeldetag: 24.03.2010
(51) Int. Cl.: A61K 9/20, A61K 31/4365, A61K 47/48

(54) **Pharmazeutische Zusammensetzung des Prasugrels**

(71) Anmelder: Ratiopharm GmbH, 89079 Ulm (DE)
(72) Erfinder: Strohmeyer, Jutta, 81476 München (DE); Meergans, Dominique, 81477 München (DE); Holfinger, Konstantin, 88326 Aulendorf (DE); Brück, Sandra, 85570 Ottenhofen (DE)
(74) Vertreter: Teipel, Stephan

(57) **Zusammenfassung**

Die vorliegende Erfindung betrifft pharmazeutische Zusammensetzungen, die Prasugrel als Wirkstoff enthalten. Die vorliegende Erfindung betrifft ferner ein Verfahren zur Herstellung dieser Zusammensetzungen.

## Beschreibung

Die vorliegende Erfindung betrifft pharmazeutische Zusammensetzungen, die Prasugrel als Wirkstoff enthalten. Die vorliegende Erfindung betrifft ferner ein Verfahren zur Herstellung dieser Zusammensetzungen.

Prasugrel hat den chemischen Namen 2-Acetoxy-5-(a-cyclopropylcarbonyl-2-fluorbenzyl)-4,5,6,7-tetrahydrothieno[3,2-c]pyridin. Prasugrel hat die folgende Strukturformel:

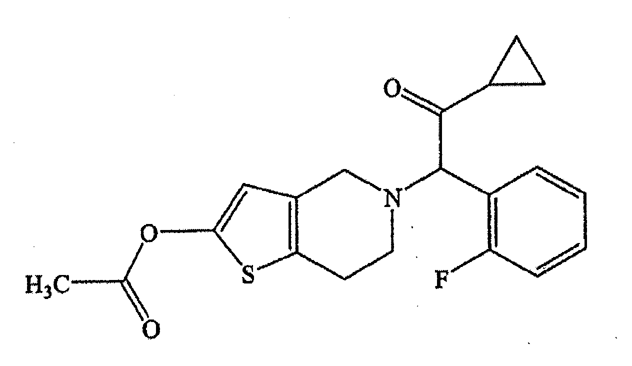

Ein Verfahren zur Herstellung von Prasugrel wird in der EP-A-0 542 411 offenbart.

Prasugrel wird als Plättchenaggregationshemmer oral verabreicht. Problematisch hierbei ist, dass es sich bei Prasugrel um einen schwer löslichen Wirkstoff handelt. Die freie Base des Wirkstoffs weist eine Löslichkeit von 57 µg/ml in Wasser auf, aber auch die Salze zeigen nur eine begrenzte Löslichkeit.

Die Löslichkeit eines Wirkstoffs lässt sich oftmals durch Mikronisation der Wirkstoffteilchen erhöhen. Aufgrund der starken Oxidationsempfindlichkeit von Prasugrel ist eine direkte Trockenvermahlung im µm-Bereich jedoch nicht möglich, da hierbei bedingt sowohl durch den mechanischen als auch thermischen Einfluss kombiniert mit der Oberflächenvergrößerung der chemische Abbau zu hoch ist.

Darüber hinaus weist Prasugrel auch einen stark pH-abhängigen Abbau auf. Bei pH-Werten von < 3 ist ein Abbau von mehr als 1 % nach bereits 2 Stunden zu beobachten. Dies schränkt die möglichen Hilfsstoffe bei der Herstellung pharmazeutischer Formulierungen ein.

Im Stand der Technik wurden verschiedene Vorschläge gemacht, die Löslichkeit von Prasugrel aus pharmazeutischen Zusammensetzungen zu verbessern und die Lagerstabilität entsprechender pharmazeutischer Zusammensetzungen zu erhöhen. So schlägt die WO 2008/072532 vor, Prasugrel zusammen mit wasserlöslichen Polymeren zu pharmazeutischen Zusammensetzungen zu verarbeiten.

Die EP-A-1 298 132 schlägt Prasugrel-Maleat und Prasugrel-Hydrochlorid als pharmazeutisch verträgliche Salze des Prasugrels und deren Verwendung zur Herstellung pharmazeutischer Zusammensetzungen vor. Sulfonsäuresalze des Prasugrels sind aus der WO 2009/129983 bekannt. Auch wenn diese Säureadditionssalze eine verbesserte Lagerstabilität aufweisen, besteht weiterhin ein Bedürfnis nach Formen des Prasugrels, die sich leicht zu pharmazeutischen Zusammensetzungen verarbeiten lassen.

Die WO 2009/130289 offenbart ein neues kristallines Polymorph von Prasugrel-Hydrogensulfat. Dieses Salz weist jedoch den Nachteil auf, dass es sich hydrolytisch zersetzt, sodass pharmazeutische Produkte, die dieses Salz enthalten, möglichst trocken und unter Inertgas gelagert werden müssen.

Darüber hinaus besteht bei den sauren Salzen des Prasugrels, und insbesondere dem Hydrochlorid und dem Hydrogensulfat, das Problem, dass diese Salzformen nicht in ausreichendem Maße stabil sind, sondern in gewissem Umfang die eingesetzten Säuren freisetzen. Dies wiederum kann zu Problemen bei der Verarbeitung sowie zu unerwünschten Reaktionen mit den bei der Herstellung der pharmazeutischen Zusammensetzungen eingesetzten Hilfsstoffe führen.

Um die Lagerstabilität Prasugrel-haltiger Tabletten zu erhöhen, schlägt die WO 2008/073759 vor, diese in einem luft- und feuchtigkeitsundurchlässigen Behälter unter einem positiven Flüssiggasdruck zu verpacken.

Es besteht somit weiterhin ein Bedürfnis nach Prasugrel-haltigen Formulierungen, die den Wirkstoff in einer möglichst löslichen Form zur Verfügung stellen. Dabei besteht auch ein Bedürfnis nach einer Verbesserung der Lösungsgeschwindigkeit sowie der Bioverfügbarkeit des Wirkstoffs. Außerdem ist es wünschenswert, die chemische Stabilität des Wirkstoffs zu verbessern, insbesondere gegen oxidativen und hydrolytischen Abbau sowie bei thermischer Belastung. Ein weiteres Problem besteht in der Verbesserung der Verarbeitbarkeit von Prasugrel zu pharmazeutischen Zusammensetzungen, die Verringerung der Hygroskopie der Zusammensetzung, sowie die Verbesserung der Lagerstabilität entsprechender Zusammensetzungen.

Es wurde nun überraschend gefunden, dass die vorstehend genannten Probleme teilweise oder ganz dadurch überwunden werden können, dass Prasugrel an ein Ionenaustauscherharz angebunden wird. Durch die Anbindung des Wirkstoff an das Ionenaustauscherharz wird der Wirkstoff überraschenderweise chemisch stabilisiert. Entsprechende Verbindungen zeigen eine geringe Hygroskopizität, eine hervorragende invitro-Freisetzung und lassen sich in chemisch großer Reinheit herstellen. Zudem ist die Fließfähigkeit des erhaltenen Reaktionsprodukts vergleichbar mit derjenigen des eingesetzten Ionenaustauscherharzes, sodass das Reaktionsprodukt leicht zu pharmazeutischen Zusammensetzungen wie Tabletten weiterverarbeitet werden kann. Es hat sich überraschend gezeigt, dass der Wirkstoff im Gastrointestinaltrakt des Patienten wieder freigesetzt wird. Das dabei ebenfalls freiwerdende Ionenaustauscherharz wird vom Körper nicht aufgenommen.

Die vorliegende Erfindung betrifft somit eine pharmazeutische Zusammensetzung, die ein Ionenaustauscherharz und daran gebundenes Prasugrel umfasst. Der Wirkstoff kann dabei in Form seiner freien Base oder als ein pharmazeutisch verträgliches Salz des Prasugrels vorliegen. Vorzugsweise ist der Wirkstoff in Form seiner freien Base an dem Ionenaustauscherharz gebunden.

Vorliegend wird unter "Wirkstoff' Prasugrel oder ein pharmazeutisch verträgliches Salz davon verstanden. Als pharmazeutisch verträgliche Salze eignen sich beispielsweise das Hydrochlorid, das Hydrobromid, das Sulfat, das Phosphat, Alkylsulfonsäuresalze, wie Methansulfonat, Trifluormethansulfonat und Ethansulfonat, Arylsulfonsäuresalze, wie Benzolsulfonat, p-Toluolsulfonat, 1-Naphthalinsulfonat, 2-Naphthalinsulfonat und 1,5-Naphthalindisulfonat, sowie Salze organischer Säuren, wie Acetat, Malat, Fumarat, Succinat, Citrat, Ascorbat, Tartrat, Oxalat und Maleat.

Mit der Bezeichnung "gebunden" wird im Sinne dieser Erfindung jede Wechselwirkung zwischen dem Wirkstoff und dem lonentauscherharz verstanden. Hierbei kann es sich beispielsweise um ionische Wechselwirkungen, π-π-Wechselwirkungen oder Wechselwirkungen aufgrund von van-der-Waals-Kräften handeln. Bevorzugt handelt es sich um Wechselwirkungen aufgrund von van-der-Waals-Kräften.

Als Ionenaustauscherharz eignet sich jedes dem Fachmann bekannte Ionenaustauscherharz, das pharmazeutisch verträglich ist und in der Lage ist, den Wirkstoff Prasugrel reversibel zu binden. Vorzugsweise handelt es sich bei dem Ionenaustauscherharz um ein Kationenaustauscherharz.

Bekannte Ionenaustauscherharze, die für die vorliegende Erfindung geeignet sind, weisen eine wasserlösliche oder wasserunlösliche Polymermatrix auf, die über sauere funktionelle Gruppen verfügt. Vorzugsweise ist die Polymermatrix wasserunlöslich.

Geeignete Ionenaustauscherharze enthalten funktionelle Gruppen, die eine Bindung mit dem Wirkstoff ermöglichen. Geeignete funktionelle Gruppen sind insbesondere schwach oder stark saure funktionelle Gruppen, wobei sich Carbonsäure- und Sulfonsäuregruppen besonders eigenen. Die funktionellen Gruppen können mit dem Wirkstoff entweder in ihrer protonierten oder deprotonierten Form in Kontakt gebracht werden. Vorzugsweise wird das Ionenaustauscherharz mit dem Wirkstoff in deprotonierter Form in Kontakt gebracht, wobei die funktionellen Gruppen dann insbesondere als deren Alkalimetallsalze, besonders bevorzugt als deren Natrium- oder Kaliumsalze eingesetzt werden.

Um einen ausreichenden Kontakt zwischen Ionenaustauscherharz und Wirkstoff zu gewährleisten, reicht ein Vermischen der Harzpartikel mit Wirkstoffpartikeln nicht aus. In der Regel ist ein Kontaktieren auf molekularer Ebene notwendig, beispielsweise durch Kontaktieren einer Harzpartikeldispersion mit darin gelösten Wirkstoffmolekülen.

Die Beladung des Ionenaustauscherharzes mit dem Wirkstoff kann entweder an der Oberfläche der Harzpartikel erfolgen oder in deren Poren. Für eine schnell freisetzende pharmazeutische Zusammensetzung ist eine Beladung mit dem Wirkstoff an der Oberfläche der Harzpartikel bevorzugt.

Für den Fall, dass die Polymermatrix des Ionenaustauscherharzes wasserlöslich ist, liegt das mittlere Molekulargewicht des Polymers vorzugsweise im Bereich von 5000 bis 1 Millionen g/mol, besonders bevorzugt im Bereich von 10.000 bis 100.000 g/mol.

Die Partikelgröße des eingesetzten Ionenaustauscherharzes ist nicht besonders eingeschränkt. Beispielsweise kann das Ionenaustauscherharz in kommerzieller Form eingesetzt werden, ohne dass es notwendig ist, die Partikelgröße beispielsweise durch Vermahlung und/oder Sieben speziell einzustellen. Beispielsweise liegt die Partikelgröße des eingesetzten Ionenaustauscherharzes zu 25 bis 30 % im Bereich von 25 µm bis 150 µm, vorzugsweise im Bereich von 75 µm bis 150 µm. Üblicherweise weisen weniger als 1 Gew.-% des eingesetzten Ionenaustauscherharzes eine Partikelgröße über 150 µm auf und mehr als 65 % liegen unter 75 µm

Als Polymermatrix des Ionenaustauscherharzes eigenen sich beispielsweise quervernetzte Styrol-Divinylbenzol- oder quervernetzte Methacrylsäure-Divinylbenzol-Copolymere. Quervernetzte Metacrylsäure-Divinylbenzol-Copolymere sind bevorzugt. Beispielsweise eignen sich die im Handel erhältlichen Ionenaustauscherharze der Amberlite^{®}-Serie, wie z.B. IRP69, IRP64 und IRP88, insbesondere Amberlite^{®} IRP88.

Das Äquivalentgewicht, d.h. die Molzahl der funktionellen Gruppen pro Gewichtseinheit des Ionenaustauscherharzes (meq/g) ist nicht besonders eingeschränkt und kann vom Fachmann frei gewählt werden. Vorzugsweise weist das eingesetzte Ionenaustauscherharz ein Äquivalentgewicht im Bereich von 1-15 meq/g, mehr bevorzugt von 2-12 meq/g und ganz besonders bevorzugt von 4-10 meq/g auf.

Der Beladungsgrad des Ionenaustauscherharzes berechnet als das Verhältnis von tatsächlich gebundenem Prasugrel zu dem maximal gebunden Prasugrel, d.h. der Menge an Prasugrel, die das Ionenaustauscherharz maximal binden kann, ist ebenfalls vom Fachmann frei zu wählen. Um eine hohen Wirkstoffanteil in der pharmazeutischen Zusammensetzung zu gewährleisten, sollte der Beladungsgrad jedoch möglichst hoch sein. Beispielsweise kann der Beladungsgrad, also das Verhältnis von tatsächlich gebundenem Prasugrel zu maximal gebundenem Prasugrel im Bereich von 1:5 bis 1:1 liegen, mehr bevorzugt im Bereich von 1:3 bis 1:1, wie beispielsweise etwa 1:2 oder etwa 1:1. Am bevorzugtesten ist der Beladungsgrad etwa 1:1.

Die erfindungsgemäße pharmazeutische Zusammensetzung kann weitere übliche pharmazeutisch verträgliche Hilfsstoffe enthalten. Bei der fertigen Darreichungsform kann es sich zum Beispiel um Tabletten, Kapseln, Sachets oder Pulver handeln. Vorzugsweise liegt die erfindungsgemäße pharmazeutische Zusammensetzung in Form einer Tablette, insbesondere einer direkt verpressten Tablette vor.

Die pharmazeutische Zusammensetzung kann zusätzlich zum Ionenaustauscherharz einen oder mehrere weitere pharmazeutisch akzeptable Hilfsstoffe enthalten, wie z.B. Füllmittel, Gleitmittel, Fließregulierungsmittel, Trennmittel, Sprengmittel, Stabilisatoren/Antioxidantien und oberflächenaktive Hilfsstoffe (Lexikon der Hilfsstoffe für Pharmazie, Kosmetik und angrenzende Gebiete", herausgegeben von H. P. Fiedler, 4. Auflage, und "Handbook of Pharmaceutical Excipients", 3. Auflage, herausgegeben von Arthur H. Kibbe, American Pharmaceutical Association, Washington, USA und Pharmaceutical Press, London).

Füllmittel: Die pharmazeutische Zusammensetzung kann ein oder mehrere Füllmittel enthalten. Im Allgemeinen handelt es sich bei einem Füllmittel um eine Substanz die das Bulk-Volumen der Mischung und somit die Größe der resultierenden Arzneiform vergrößert. Bevorzugte Beispiele für Füllmittel sind Lactose, mikrokristalline Cellulose (z.B. Avicel) und Calciumhydrogenphosphat. Das Füllmittel kann einen Anteil von 0 bis 90 Gew.-%, bevorzugt zwischen 25 und 85 Gew.-% des Gesamtgewichts der Zusammensetzung haben.

Gleitmittel: Die Funktion des Gleitmittels ist es, sicherzustellen, dass die Tablettenpressung und der Auswurf ohne große Reibung zwischen den Festoffen und den Wandungen erfolgt. Das Gleitmittel ist vorzugsweise ein Erdalkalimetallstearat, wie Magnesiumstearat oder eine Fettsäure, wie Stearinsäure. Das Gleitmittel ist üblicherweise in einer Menge von 0 bis 2 Gew.-%, bevorzugt zwischen 0,5 und 1,5 Gew.-% des Gesamtgewichts der pharmazeutischen Zusammensetzung vorhanden.

Sprengmittel: Gewöhnlich wird unter einem Sprengmittel eine Substanz verstanden, die in der Lage ist, die Tablette in kleinere Teile aufzubrechen, sobald sie in Kontakt mit einer Flüssigkeit ist. Bevorzugte Sprengmittel sind Croscarmellose Natrium, Natriumcarboxymethylstärke, quervernetztes Polyvinylpyrrolidon (Crospovidon) oder Natriumcarboxymethylglycolat (z.B. Explotab) und Natriumbicarbonat. Das Sprengmittel ist normalerweise in einem Anteil von 0 bis 20 Gew.-%, bevorzugt zwischen 1 und 15 Gew.-% des Gesamtgewichts der Zusammensetzung vorhanden.

Fließregulierungsmittel: Als Fließregulierungsmittel kann z.B. kolloidales Siliziumdioxid verwendet werden. Bevorzugt ist das Fließregulierungsmittel in einer Menge von 0 bis 8 Gew.-%, mehr bevorzugt in einer Menge zwischen 0.1 und 3 Gew.-% des Gesamtgewichts der Zusammensetzung vorhanden.

Stabilisatoren/Antioxidantien: Als Stabilisatoren und Antioxidantien können Ascorbinsäure und Acorbate, EDTA und seine Salze, Citronensäure und Citrate, Butylhydroxyanisol, Butylhydroxytoluol oder Vitamin E verwendet werden. Bevorzugt wird Vitamin E als Stabilisator verwendet.

Oberflächenaktive Hilfsstoffe: Als oberflächenaktive Hilfsstoffe können anionische Tenside wie z.B. Natriumlaurylsulfat, Natriumcetylstearylsulfat und Natriumdioctylsulfosuccinat, amphotere Tenside wie z. B. Lecithin und nichtionische Tenside wie z.B. Cetylakohol, Stearylalkohol, Sorbitanfettsäureester, Polyoxyethylen-Sorbitanfettsäureester, Polyoxyethylen-Fettsäureglyceride, Polyoxyethylen-Fettsäureester, Polyoxyethylen-Fettalkoholether und Glycerolfettsäureester verwendet werden. Bevorzugt ist die Verwendung von anionischen Tensiden und ganz besonders bevorzugt die Verwendung von Natriumlaurylsulfat.

Die vorliegende Erfindung betrifft darüber hinaus ein Verfahren zur Herstellung der vorstehend beschriebenen pharmazeutischen Zusammensetzung. Das Verfahren umfasst zunächst den Schritt des Lösens von Prasugrel oder einem pharmazeutisch verträglichen Salz davon in einem Lösungsmittel. In dieser Lösung wird der Wirkstoff mit dem Ionenaustauscherharz in Kontakt gebracht. Dabei kann das Ionenaustauscherharz entweder zuvor in dem Lösungsmittel suspendiert vorgelegen haben oder nach dem Lösen des Wirkstoffs zugegeben worden sein. Ionenaustauscherharz und Wirkstoff können auch gleichzeitig in das Lösungsmittel gegeben werden. Nach Abschluss der Reaktion wird das geladene Ionenaustauscherharz aus der Lösung beispielsweise durch Abfiltrieren gewonnen und gewaschen.

Das Kontaktieren zwischen Ionenaustauscherharz und Wirkstoff erfolgt vorzugsweise bei Raumtemperatur unter Rühren. Als Wirkstoff kann entweder die freie Base des Prasugrels oder ein Salz, insbesondere ein pharmazeutisch verträgliches Salz des Prasugrels eingesetzt werden. Besonders bevorzugt wird Prasugrel-Hydrochlorid eingesetzt.

Als Lösungsmittel kann jedes Lösungsmittel verwendet werden, das eine ausreichende Löslichkeit für den Wirkstoff aufweist. Beispielsweise eignen sich polare, protische oder unprotische organische Lösungsmittel oder Wasser. Auch Gemische aus einem organischen, mit Wasser mischbaren Lösungsmittel und Wasser können eingesetzt werden. Als organische Lösungsmittel eignen sich beispielsweise Alkohole, wie Butanol, Propanol, Isopropanol, Ethanol und Methanol. Bevorzugt wird Ethanol oder Ethanol/Wasser Gemisch verwendet.

Die Konzentration des Wirkstoffs im Reaktionsmedium kann zwischen 0,1 und 5 Gew.-%, vorzugsweise zwischen 0,3 und 1 Gew.-% bezogen auf die Gesamtmenge aus Ionenaustauscherharz, Wirkstoff und Lösungsmittel liegen.

Der Reaktionsfortschritt kann durch eine Veränderung des pH-Wertes der Lösung beobachtet werden. Zusätzlich kann auch der Wirkstoffgehalt in der Reaktionslösung gemessen werden, beispielsweise durch HPLC. Diese Methode kann auch zur Bestimmung der Beladung des Harzes verwendet werden.
Figur 1 zeigt von oben nach unten die Röntgenpulverdiffraktogramme der erfindungsgemäßen Zusammensetzung aus Beispiel 1, von Prasugrel freie Base und von Prasugrel Hydrochlorid.
Figur 2 zeigt von oben nach unten die IR-Spektren von Prasugrel Hydrochlorid und der erfindungsgemäßen Zusammensetzung aus Beispiel 1.
Figur 3 zeigt von oben nach unten die DSC-Thermogramme von Prasugrel Hydrochlorid, der erfindungsgemäßen Zusammensetzung aus Beispiel 1 und Prasugrel freie Base.

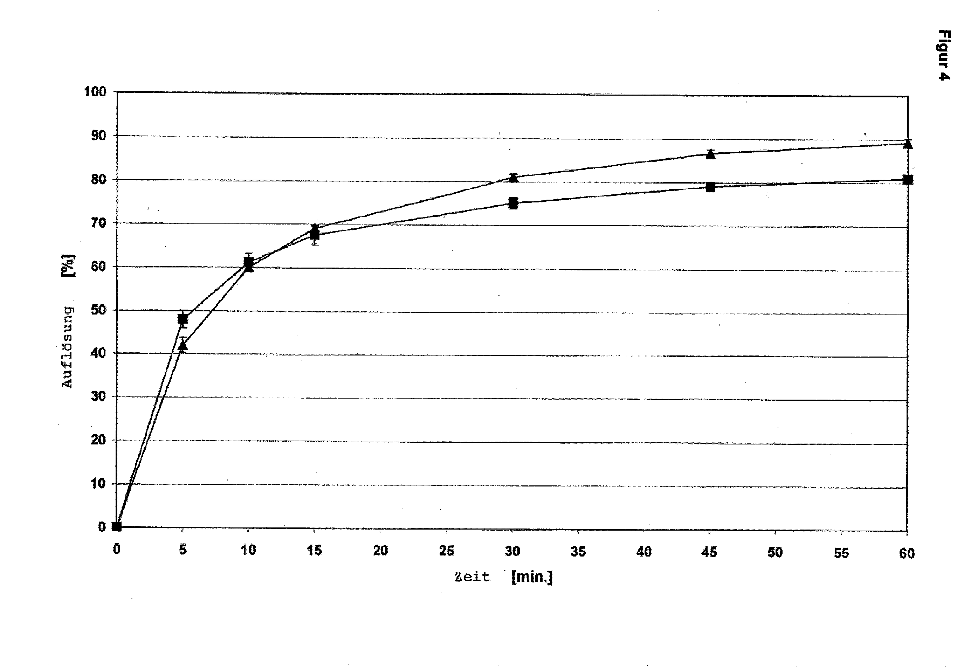

Figur 4 zeigt das Freisetzungsprofil der erfindungsgemäßen pharmazeutischen Zusammensetzung aus Beispiel 2a (Dreiecke) im Vergleich zum Freisetzungsprofil des kommerziellen Prasugrel-Produkts Efient^{®} (Vierecke).

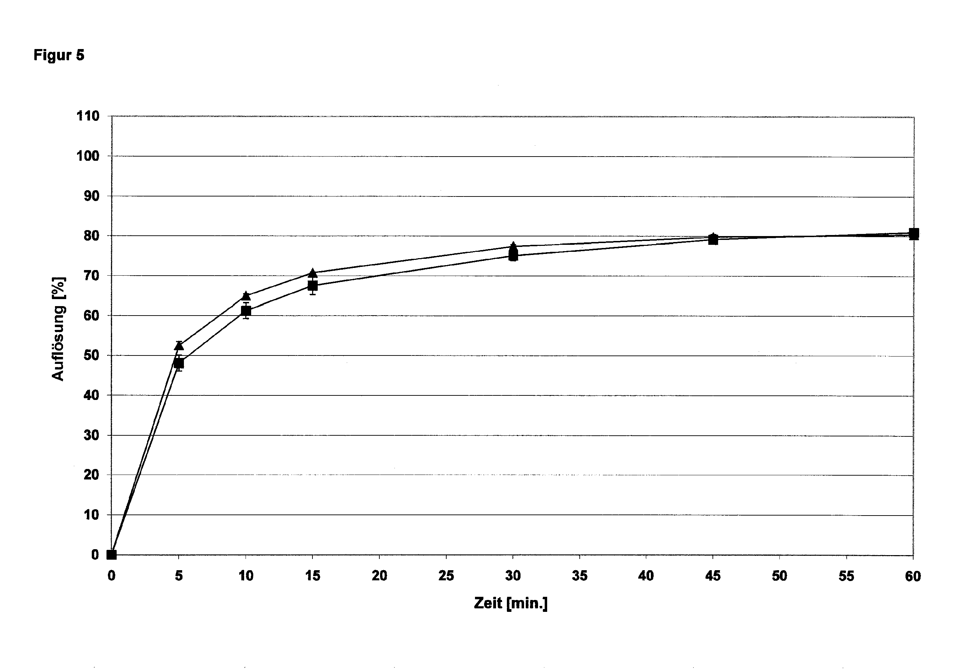

Figur 5 zeigt das Freisetzungsprofil der erfindungsgemäßen pharmazeutischen Zusammensetzung aus Beispiel 2b (Dreiecke) im Vergleich zum Freisetzungsprofil des kommerziellen Prasugrel-Produkts Efient^{®} (Vierecke).

Die vorliegende Erfindung wird nun anhand der nachfolgenden Beispiele näher erläutert, ohne dass diese einschränkend ausgelegt werden sollen.

### Beispiel 1

Prasugrel HCI (200 mg) wird unter Rühren in einem Ethanol/demineralisiertes WasserGemisch (150 ml/100 ml) gelöst. Amberlite^{®} IRP88 (200 mg) wird in einem Massenverhältnis von 1:1 (HCI-Salz zu Ionenaustauscherharz) und bei Raumtemperatur mit dem Wirkstoff zur Reaktion gebracht. Der Reaktionsfortschritt wird durch Messung des pH-Werts überwacht.

Wenn sich der pH-Wert nicht mehr ändert, wird das Reaktionsprodukt abfiltriert, mit demineralisiertem Wasser gewaschen und bei ca. 70°C getrocknet (14 h).

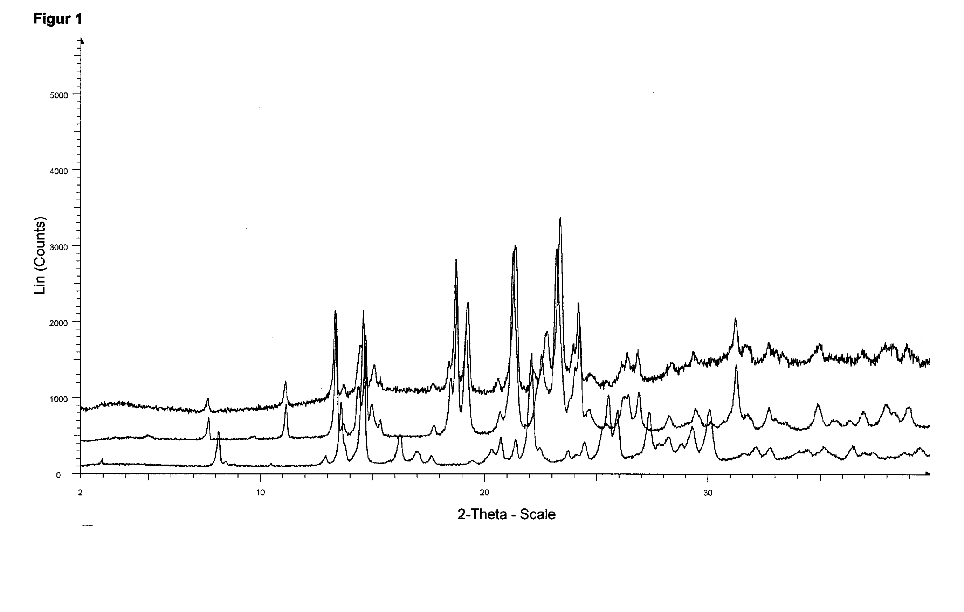

Aus dem Röntgenpulverdiffraktogramm der in diesem Beispiel erhaltenen Zusammensetzung (oberstes Diffraktogramm in Figur 1) erkennt man, dass der Wirkstoff in der erfindungsgemäßen Zusammensetzung nicht mehr als Hydrochlorid (unterstes Diffraktogramm in Figur 1) vorliegt. Das eingesetzte Prasugrel Hydrochlorid ist somit in der Dispersion des Ionenaustauscherharzes eine Reaktion eingegangen und liegt in der erhaltenen Zusammensetzung an dem Ionenaustauscherharz gebunden vor. Das Röntgenpulverdiffraktogramm der erfindungsgemäßen Zusammensetzung entspricht dabei im Wesentlichen demjenigen von Prasugrel freie Base (mittleres Diffraktogramm in Figur 1). Das an dem Ionenaustauscherharz gebundene Prasugrel liegt somit nicht mehr in Form des eingesetzten Prasugrel Hydrochlorids vor.

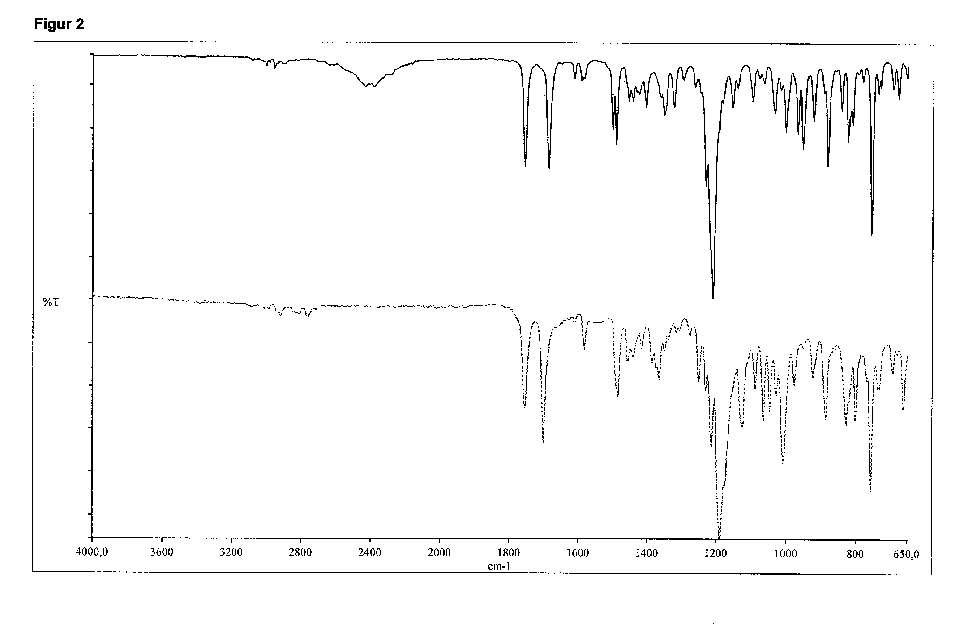

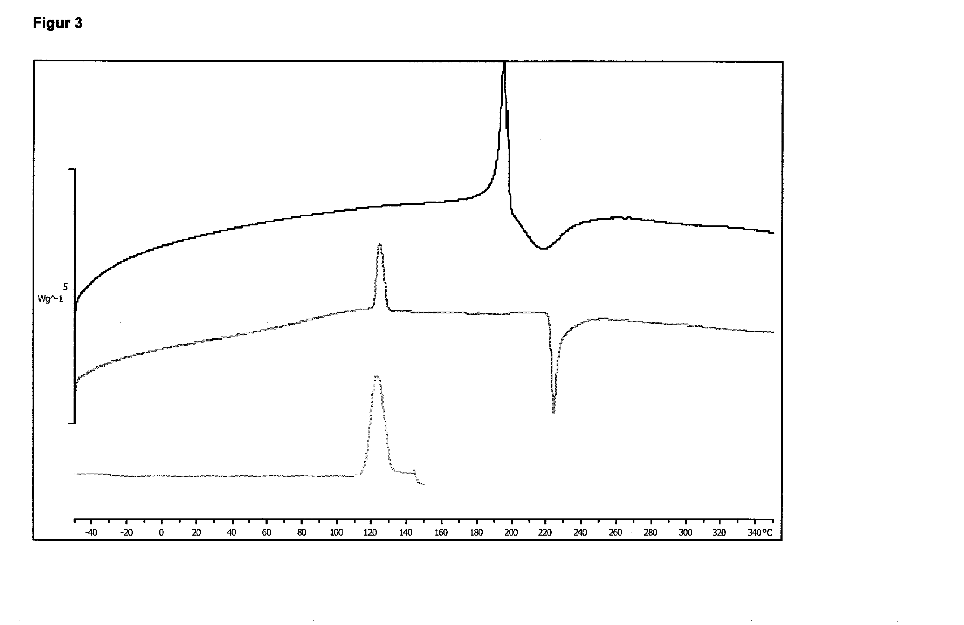

Die Tatsache, dass in der erfindungsgemäßen Zusammensetzung der Wirkstoff nicht mehr in Form des eingesetzten Prasugrel Hydrochlorids vorliegt, wird auch durch die anliegenden IR-Spektren (Figur 2) sowie die DSC-Thermogramme (Figur 3) bestätigt.

### Beispiel 2a

Tablette folgender Zusammensetzung wurde hergestellt

| | |
|---|---|
| Zusammensetzung aus Beispiel 1 | 10,87 mg |
| Natriumcroscarmellose (Acdisol) | 22,00 mg |
| mikrokristalline Cellulose (Avicel) | 80,00 mg |
| Natriumlaurylsulfat (SDS) | 25,00 mg |
| | |
| Gesamt | 137,87 mg |

Die Inhaltsstoffe werden im Freifallmischer gemischt und anschließend zu Tabletten verpresst.

### Beispiel 2b:

Tablette folgender Zusammensetzung wurde hergestellt

| | |
|---|---|
| Zusammensetzung aus Beispiel 1 | 10,87 mg |
| Natriumcroscarmellose (Acdisol) | 12,00 mg |
| mikrokristalline Cellulose (Avicel) | 100,00 mg |
| Natriumlaurylsulfat (SDS) | 3,20 mg |
| | |
| Gesamt | 126,07 mg |

Das Resinat-Produkt aus Beispiel 1 wird dabei zunächst im Mörser zerrieben und danach werden die restlichen Hilfsstoffe zugegeben. Die Inhaltsstoffe werden im Freifallmischer gemischt und anschließend zu Tabletten verpresst.

### Beispiel 3

Die Stabilität der in Beispiel 2a erhaltenen Tabletten wurde bestimmt. Die Tabletten wurden dazu bei 40°C/75% rel. Luftfeuchtigkeit für bis zu 8 Wochen gelagert. Die Ergebnisse sind in der folgenden Tabelle zusammengefasst.

| **Zeitpunkt** | **Anfang** | **4 Wochen** | **8 Wochen** |
|---|---|---|---|
| Beispiel 2a Zersetzung (%) | 0,31 | 0,42 | 0,47 |
| Efient^{®} Zersetzung (%) | 0,47 | 0,55 | 0,78 |

Die vorstehenden Ergebnisse der Stabilitätsmessung belegen die ausgezeichnete Lagerstabilität der erfindungsgemäßen pharmazeutischen Zusammensetzung selbst unter hoher relativer Luftfeuchtigkeit.

### Beispiel 4

Die Freisetzungsprofile der in den Beispielen 2a und 2b erhaltenen Tabletten sowie des kommerziellen Produkts Efient^{®} wurde in 900 ml 50 mM Acetatpuffer (pH 4,5) bei 37°C und 75 UpM nach der USP-Methode (App. II) gemessen.

Das gemessene Freisetzungsprofil der erfindungsgemäßen Tabletten des Beispiels 2a (Dreiecke) ist in Figur 1 zusammen mit dem Freisetzungsprofil des kommerziellen Prasugrel-Produkts Efient^{®} (Vierecke) dargestellt. In Figur 2 ist das Freisetzungsprofil der erfindungsgemäßen Tabletten des Beispiels 2b (Dreiecke) zusammen mit dem Freisetzungsprofil des kommerziellen Prasugrel-Produkts Efient^{®} (Vierecke) dargestellt. Man erkennt, dass die erfindungsgemäßen Zusammensetzungen im Vergleich zu dem kommerziellen Prasugrel-Produkt eine schnellere Freisetzung des Wirkstoffs zur Verfügung stellt.

## Patentansprüche

1. Pharmazeutische Zusammensetzung, umfassend ein Ionenaustauscherharz und daran gebundenes Prasugrel.

2. Pharmazeutische Zusammensetzung nach Anspruch 1, worin das Ionenaustauscherharz ein Kationenaustauscherharz ist.

3. Pharmazeutische Zusammensetzung nach Anspruch 1 oder 2, worin das Ionenaustauscherharz über eine wasserlösliche oder wasserunlösliche Polymermatrix mit sauren funktionellen Gruppen verfügt.

4. Pharmazeutische Zusammensetzung nach Anspruch 3, worin die sauren funktionellen Gruppen ausgewählt sind aus Carbonsäure- und Sulfonsäuregruppen.

5. Pharmazeutische Zusammensetzung nach Anspruch 4, worin die Carbonsäure-oder Sulfonsäuregruppen deprotoniert sind und als deren Alkalimetallsalze, insbesondere Natrium- oder Kaliumsalze mit dem Prasugrel zur Reaktion gebracht wurden.

6. Pharmazeutische Zusammensetzung nach einem der vorhergehenden Ansprüche, worin das Ionenaustauscherharz eine Partikelgröße im Wesentlichen im Bereich von 25 µm bis 150 µm aufweist.

7. Pharmazeutische Zusammensetzung nach einem der vorhergehenden Ansprüche, worin das Ionenaustauscherharz eine Polymermatrix aus einem quervernetzten Methacrylsäure-Divinylbenzol-Copolymer aufweist.

8. Pharmazeutische Zusammensetzung nach einem der vorhergehenden Ansprüche, worin die Molzahl der funktionellen Gruppen pro Gramm des Ionenaustauscherharzes (meq/g) im Bereich von 1-15 meq/g liegt.

9. Pharmazeutische Zusammensetzung nach einem der vorhergehenden Ansprüche, worin der Beladungsgrad des Ionenaustauscherharzes berechnet als Verhältnis von tatsächlich gebundenem Prasugrel zu maximal gebundenem Prasugrel im Bereich von 1:5 bis 1:1 liegt.

10. Pharmazeutische Zusammensetzung nach einem der vorhergehenden Ansprüche, die als Tablette, vorzugsweise direkt verpresste Tablette, vorliegt.

11. Verfahren zur Herstellung einer pharmazeutischen Zusammensetzung nach einem der Ansprüche 1-10, umfassend das Lösen von Prasugrel oder einem Salz davon in einem Lösungsmittel, das Kontaktieren des gelösten Prasugrels mit dem Ionenaustauscherharz und das Gewinnen des erhaltenen Reaktionsprodukts.
